# EUROPÄISCHE PATENTANMELDUNG

(11) **EP 0 767 378 A1**
(43) Veröffentlichungstag der Anmeldung: **09.04.1997**
(21) Anmeldenummer: 96113179.4
(22) Anmeldetag: 15.06.1994
(51) Int. Cl.: G01N 33/574, C12P 21/08, C07K 16/28, G01N 33/577, A61K 49/00

(54) **Verfahren zur Diagnose und Analyse von Magenkarzinomen**

(30) Priorität: 22.06.1993 DE 4320623; 22.06.1993 DE 4320624; 02.07.1993 DE 4321944; 28.04.1994 DE 4414787
(62) Teilanmeldung aus: 94919633.1
(71) Anmelder: BOEHRINGER INGELHEIM INTERNATIONAL GmbH, D-55216 Ingelheim am Rhein (DE); Forschungszentrum Karlsruhe GmbH, 76021 Karlsruhe (DE)
(72) Erfinder: Ponta, Helmut, Prof. Dr., 76351 Linkenheim-Hochstetten (DE); Herrlich, Peter, Prof. Dr., 76229 Karlsruhe (41) (DE); Heider, Karl-Heinz, Dr. Dipl.-Biol., 1121 Wien (AT)

(57) **Zusammenfassung**

Die Erfindung bertifft ein Verfahren zur Diagnose und Analyse von Magenkarzinomen, das auf dem Nachweis der Expression bestimmter varianter Exons des CD44-Gens beruht. Der Nachweis kann auf Protein- oder Nukleinsäureebene erfolgen. In einer bevorzugten Ausführungsform wird die Expression in Biopsiematerial mit Hilfe exonspezifischer Antikörper nachgewiesen.

SEQUENZPROTOKOLL

(1) ALLGEMEINE ANGABEN:

(i) ANMELDER:
(A) NAME: Boehringer Ingelheim International GmbH
(B) STRASSE: Binger Strasse
(C) ORT: Ingelheim
(E) LAND: Deutschland
(F) POSTLEITZAHL: 55218
(G) TELEFON: 49-6132-772770
(H) TELEFAX: 49-6132-4473

(A) NAME: Forschungszentrum Karlsruhe GmbH
(B) STRASSE: Postfach 3640
(C) ORT: Karlsruhe
(E) LAND: Deutschland
(F) POSTLEITZAHL: 76021

(ii) BEZEICHNUNG DER ERFINDUNG: Verfahren zur Diagnose und Analyse von
Magenkarzinomen

(iii) ANZAHL DER SEQUENZEN: 14

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 120 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:
(A) NAME/SCHLÜSSEL: CDS
(B) LAGE:3..119

(ix) MERKMAL:
(A) NAME/SCHLÜSSEL: exon
(B) LAGE:1..120

(xi) SEQUENZBESCHREIBUNG: SEQ ID NO: 1:

CA GAT GTA GAC AGA AAT GGC ACC ACT GCT TAT GAA GGA AAC TGG AAC 47
Asp Val Asp Arg Asn Gly Thr Thr Ala Tyr Glu Gly Asn Trp Asn
1               5                   10                  15

CCA GAA GCA CAC CCT CCC CTC ATT CAC CAT GAG CAT CAT GAG GAA GAA 95
Pro Glu Ala His Pro Pro Leu Ile His His Glu His His Glu Glu Glu
                20                  25                  30

GAG ACC CCA CAT TCT ACA AGC ACA A 120
Glu Thr Pro His Ser Thr Ser Thr

            35

(2) ANGABEN ZU SEQ ID NO: 2:

(i) SEQUENZKENNZEICHEN:
(A) LÄNGE: 39 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 2:

Asp Val Asp Arg Asn Gly Thr Thr Ala Tyr Glu Gly Asn Trp Asn Pro
1               5                   10                  15

Glu Ala His Pro Pro Leu Ile His His Glu His His Glu Glu Glu Glu
            20                  25                  30

Thr Pro His Ser Thr Ser Thr
        35

(2) ANGABEN ZU SEQ ID NO: 3:

(i) SEQUENZKENNZEICHEN:
(A) LÄNGE: 129 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:
(A) NAME/SCHLÜSSEL: CDS
(B) LAGE:3..128

(ix) MERKMAL:
(A) NAME/SCHLÜSSEL: exon
(B) LAGE:1..129

(xi) SEQUENZBESCHREIBUNG: SEQ ID NO: 3:

TC CAG GCA ACT CCT AGT AGT ACA ACG GAA GAA ACA GCT ACC CAG AAG      47
   Gln Ala Thr Pro Ser Ser Thr Thr Glu Glu Thr Ala Thr Gln Lys
    40                  45                  50

GAA CAG TGG TTT GGC AAC AGA TGG CAT GAG GGA TAT CGC CAA ACA CCC      95
Glu Gln Trp Phe Gly Asn Arg Trp His Glu Gly Tyr Arg Gln Thr Pro
 55                  60                  65                  70

AGA GAA GAC TCC CAT TCG ACA ACA GGG ACA GCT G                        129
Arg Glu Asp Ser His Ser Thr Thr Gly Thr Ala
                75                  80

(2) ANGABEN ZU SEQ ID NO: 4:

(i) SEQUENZKENNZEICHEN:
(A) LÄNGE: 42 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 4:

Gln Ala Thr Pro Ser Ser Thr Thr Glu Glu Thr Ala Thr Gln Lys Glu
1               5                   10                  15

Gln Trp Phe Gly Asn Arg Trp His Glu Gly Tyr Arg Gln Thr Pro Arg
            20                  25                  30

Glu Asp Ser His Ser Thr Thr Gly Thr Ala
        35                  40

(2) ANGABEN ZU SEQ ID NO: 5:

(i) SEQUENZKENNZEICHEN:
(A) LÄNGE: 132 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:
(A) NAME/SCHLÜSSEL: CDS
(B) LAGE:3..131

(ix) MERKMAL:
(A) NAME/SCHLÜSSEL: exon
(B) LAGE:1..132

(xi) SEQUENZBESCHREIBUNG: SEQ ID NO: 5:

CA GCC TCA GCT CAT ACC AGC CAT CCA ATG CAA GGA AGG ACA ACA CCA      47
   Ala Ser Ala His Thr Ser His Pro Met Gln Gly Arg Thr Thr Pro
           45                  50                  55

AGC CCA GAG GAC AGT TCC TGG ACT GAT TTC TTC AAC CCA ATC TCA CAC      95
Ser Pro Glu Asp Ser Ser Trp Thr Asp Phe Phe Asn Pro Ile Ser His
        60                  65                  70

CCC ATG GGA CGA GGT CAT CAA GCA GGA AGA AGG ATG G                    132
Pro Met Gly Arg Gly His Gln Ala Gly Arg Arg Met
    75                  80                  85

(2) ANGABEN ZU SEQ ID NO: 6:

(i) SEQUENZKENNZEICHEN:
(A) LÄNGE: 43 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 6:

Ala Ser Ala His Thr Ser His Pro Met Gln Gly Arg Thr Thr Pro Ser
1               5                   10                  15

Pro Glu Asp Ser Ser Trp Thr Asp Phe Phe Asn Pro Ile Ser His Pro
            20                  25                  30

Met Gly Arg Gly His Gln Ala Gly Arg Arg Met

35                    40

(2) ANGABEN ZU SEQ ID NO: 7:

(i) SEQUENZKENNZEICHEN:
(A) LÄNGE: 102 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:
(A) NAME/SCHLÜSSEL: CDS
(B) LAGE:3..101

(ix) MERKMAL:
(A) NAME/SCHLÜSSEL: exon
(B) LAGE:1..102

(xi) SEQUENZBESCHREIBUNG: SEQ ID NO: 7:

AT ATG GAC TCC AGT CAT AGT ACA ACG CTT CAG CCT ACT GCA AAT CCA 47
Met Asp Ser Ser His Ser Thr Thr Leu Gln Pro Thr Ala Asn Pro
45                    50                    55

AAC ACA GGT TTG GTG GAA GAT TTG GAC AGG ACA GGA CCT CTT TCA ATG 95
Asn Thr Gly Leu Val Glu Asp Leu Asp Arg Thr Gly Pro Leu Ser Met
60                    65                    70

ACA ACG C 102
Thr Thr
75

(2) ANGABEN ZU SEQ ID NO: 8:

(i) SEQUENZKENNZEICHEN:
(A) LÄNGE: 33 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 8:

Met Asp Ser Ser His Ser Thr Thr Leu Gln Pro Thr Ala Asn Pro Asn
1               5                   10                  15

Thr Gly Leu Val Glu Asp Leu Asp Arg Thr Gly Pro Leu Ser Met Thr
20                  25                  30

Thr

(2) ANGABEN ZU SEQ ID NO: 9:

(i) SEQUENZKENNZEICHEN:
(A) LÄNGE: 78 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 9:

Ala Ser Ala His Thr Ser His Pro Met Gln Gly Arg Thr Thr Pro Ser
1               5                   10                  15

Pro Glu Asp Ser Ser Trp Thr Asp Phe Phe Asn Pro Ile Ser His Pro
            20                  25                  30

Met Gly Arg Gly His Gln Ala Gly Arg Arg Met Asp Met Asp Ser Ser
        35                  40                  45

His Ser Thr Thr Leu Gln Pro Thr Ala Asn Pro Asn Thr Gly Leu Val
    50                  55                  60

Glu Asp Leu Asp Arg Thr Gly Pro Leu Ser Met Thr Thr Gln
65                  70                  75

(2) ANGABEN ZU SEQ ID NO: 10:

(i) SEQUENZKENNZEICHEN:
(A) LÄNGE: 14 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 10:

Gln Trp Phe Gly Asn Arg Trp His Glu Gly Tyr Arg Gln Thr
1               5                   10

(2) ANGABEN ZU SEQ ID NO: 11:

(i) SEQUENZKENNZEICHEN:
(A) LÄNGE: 27 Basenpaare
(B) ART: Nucleotid
(C) STRANGFORM: Einzelstrang
(D) TOPOLOGIE: linear

(ii) ART DES MOLEKÜLS: cDNA zu mRNA

(xi) SEQUENZBESCHREIBUNG: SEQ ID NO: 11:

CAGGCTGGGA GCCAAATGAA GAAAATG                                    27

(2) ANGABEN ZU SEQ ID NO: 12:

(i) SEQUENZKENNZEICHEN:
(A) LÄNGE: 30 Basenpaare
(B) ART: Nucleotid
(C) STRANGFORM: Einzelstrang
(D) TOPOLOGIE: linear

(ii) ART DES MOLEKÜLS: cDNA zu mRNA

(xi) SEQUENZBESCHREIBUNG: SEQ ID NO: 12:

TGATAAGGAA CGATTGACAT TAGAGTTGGA 30

(2) ANGABEN ZU SEQ ID NO: 13:

(i) SEQUENZKENNZEICHEN:
(A) LÄNGE: 39 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 13:

Asp Val Asp Arg Asn Gly Thr Thr Ala Tyr Glu Gly Asn Trp Asn Pro
1               5                   10                  15

Glu Ala His Pro Pro Leu Ile His His Glu His His Glu Glu Glu Glu
            20                  25                  30

Thr Pro His Ser Thr Ser Thr
        35

(2) ANGABEN ZU SEQ ID NO: 14:

(i) SEQUENZKENNZEICHEN:
(A) LÄNGE: 42 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 14:

Gln Ala Thr Pro Ser Ser Thr Thr Glu Glu Thr Ala Thr Gln Lys Glu
1               5                   10                  15

Gln Trp Phe Gly Asn Arg Trp His Glu Gly Tyr Arg Gln Thr Pro Arg
            20                  25                  30

Glu Asp Ser His Ser Thr Thr Gly Thr Ala
        35                  40

## Beschreibung

Die Erfindung betrifft Verfahren zur Diagnose und/oder Analyse von Magenkarzinomen durch Bestimmung der Expression variabler Exons des CD44-Gens, Mittel für solche Verfahren sowie deren Verwendung.

Es besteht ein Bedarf nach verbesserten Verfahren zur Diagnose und/oder Analyse von Krebserkrankungen, insbesondere auf Grundlage von molekularen Markern.

Beispielsweise findet die hämatogene Ausbreitung von Mammakarzinomen sehr früh im Krankheitsverlauf statt und steht im Zusammenhang mit dem späteren Auftreten von Fernmetastasen (Diel *et al.,* 1992). Die molekularen Mechanismen der metastatischen Ausbreitung sind immer noch unbekannt. Die prognostischen Faktoren für die Voraussage des Metastasierungsrisikos beruhen momentan hauptsächlich auf pathologischen Kriterien, wobei die Hauptfaktoren Tumorstadium, Differenzierung (Gradierung) sowie Lymphknotenmetastasierung sind (Fisher *et al.,* 1990). Allerdings treten in Einzelfällen Diskrepanzen zwischen diesen Faktoren auf, etwa indem trotz fortgeschrittener Tumorgröße oder niedriger Differenzierung (hoher Gradierung) Lymphknotenmetastasen fehlen. Wenig untersucht ist eine Untergruppe von Patientinnen mit Lymphknoten-negativem Brustkrebs, die später Fernmetastasen entwickeln. Es besteht daher ein Bedarf an Parametern, die eine bessere Voraussage der hämatogenen Tumorausbreitung sowie einer besseren Allgemeinprognose gestatten. Ein anderes Beispiel sind Magentumoren. Sie können in zwei große histologische Kategorien eingeteilt werden, den "intestinalen" und den "diffusen" Typ (Lauren, 1965). Tumoren vom intestinalen, nicht jedoch vom diffusen Typ sind oft von einer chronischen Gastritis B und insbesondere von intestinalen Metaplasien begleitet, die für Vorläufer von dysplastischen Veränderungen und von Adenokarzinomen des intestinalen Typs gehalten werden (Jida *et* Kusama, 1982; Jass, 1983; Kato *et al.,* 1981; Sipponen *et al.,* 1983; Sirula *et al.,* 1974; Strickland *et* Mackay, 1973). Pathogenetische Unterschiede zwischen diesen beiden Adenokarzinom-Typen spiegeln sich auch in der Beobachtung wider, daß Patienten mit Tumoren vom diffusen Typ oft zur Blutgruppe A gehören, was einen möglichen Einfluß von genetischen Faktoren auf das Krebsrisiko anzeigt (Piper, 1978), während Umweltfaktoren, z.B. Infektionen mit *Helicobacter pylori,* möglicherweise für die Entwicklung von Tumoren vom intestinalen Typ wichtig sind (Parsonnet *et al.,* 1991; Nomura *et al.,* 1991). Hier wäre es wünschenswert, anhand molekularer Marker zwischen Tumoren vom intestinalen und solche vom diffusen Typ unterscheiden zu können. Als drittes Beispiel sei schließlich das Zervixkarzinom des Uterus genannt. Trotz abnehmender Inzidenz (Petterson, 1988) ist die Prognose von Patientinnen mit fortgeschrittenen Stadien von Zervixkarzinomen schlecht (Perez *et al.,* 1983; Park *et* Thigpen, 1993; Brady *et al.,* 1986). Die Frühdiagnose basiert auf der Auswertung früher morphologischer Veränderungen der Epithelzellen (zervikaler Abstrich). Auch hier ist es wünschenswert, definierte molekulare Marker zur frühen Krebserkennung, die für das Staging und als prognostische Faktoren verwendet werden können, zu finden.

Es wurde kürzlich gezeigt, daß die Expression von Varianten des Oberflächen-Glykoproteins CD44 notwendig und hinreichend ist, um sogenanntes spontanes metastatisches Verhalten sowohl in einer nicht-metastasierenden Pankreas-Adenokarzinom-Zellinie der Ratte als auch in einer nicht-metastasierenden Fibrosarkom-Zellinie der Ratte auszulösen (Günthert *et al.,* 1991). Während die kleinste CD44-Isoform, die Standardform CD44s, in einer Reihe verschiedener Gewebe, darunter Epithelzellen, ubiquitär exprimiert wird, werden bestimmte Spleißvarianten von CD44 (CD44v) nur auf einer Untergruppe von Epithelzellen exprimiert. Die CD44-Isoformen werden durch alternatives Spleißen so erzeugt, daß die Sequenzen von 10 Exons (v1-v10) in CD44s komplett ausgeschnitten werden, jedoch bei den größeren Varianten in verschiedenen Kombinationen vorkommen können (Screaton *et al.,* 1992; Heider *et al.,* 1993; Hofinann *et al.,* 1991). Die Varianten unterscheiden sich dadurch, daß an einer bestimmten Stelle des extrazellulären Teils des Proteins unterschiedliche Aminosäuresequenzen inseriert sind. Solche Varianten konnten in verschiedenen menschlichen Tumorzellen und in menschlichem Tumorgewebe nachgewiesen werden. So wurde kürzlich die Expession von CD44-Varianten im Verlauf der kolorektalen Karzinogenese untersucht (Heider *et al.,* 1993). Die Expression von CD44-Varianten fehlt in normalem menschlichem Kolonepithel, und nur eine schwache Expression ist in den proliferierenden Zellen der Krypten nachweisbar. In späteren Stadien der Tumorprogression, z.B. in Adenokarzinomen, exprimieren alle malignen Entartungen Varianten von CD44. Weiter wurde kürzlich Expression von CD44-Spleißvarianten in aktivierten Lymphozyten sowie in Non-Hodgkin-Lymphomen gezeigt (Koopman *et al.,* 1993).

Aufgabe der vorliegenden Erfindung war die Entwicklung von neuen Verfahren zur Diagnose und Analyse von Tumoren sowie die Bereitstellung von Mitteln für solche Verfahren.

Diese Aufgabe konnte mit der vorliegenden Erfindung gelöst werden. Sie betrifft ein Verfahren zur Diagnose und/oder Analyse von Tumoren, insbesondere Magenkarzinomen, das dadurch gekennzeichnet ist, daß variantes CD44 als molekularer Marker verwendet wird.

Bevorzugt werden dabei Verfahren, die auf dem Nachweis der Expression einzelner, definierter variabler Exons des CD44-Gens oder definierter Kombinationen solcher Exons beruhen. Dabei kann es vorteilhaft sein, die Expression verschiedener varianter Exons in einer Probe zu untersuchen und zueinander ins Verhältnis zu setzen.

Besonders bevorzugt sind Verfahren, die auf dem Nachweis der Expression der variablen Exons v5 und/oder v6 beruhen.

Die erfindungsgemäßen Verfahren können an Proben außerhalb des menschlichen oder tierischen Körpers oder aber *in vivo* vorgenommen werden.

Vorteilhaft können mit den erfindungsgemäßen Verfahren Proben von Patienten untersucht werden, bei denen der Verdacht auf Magenkarzinom besteht oder die Diagnose bereits vorliegt, der Tumor aber genauer charakterisiert werden soll. Insbesondere kann bei Verdacht auf/Diagnose von Magenkarzinom die Expression der Exons v5 und/oder v6 untersucht werden.

Der Nachweis varianter CD44-Moleküle kann auf Proteinebene mittels Antikörpern oder auf Nukleinsäureebene mittels spezifischer Nukleinsäuresonden oder Primern für die Polymerase-Kettenreaktion (PCR) erfolgen. Die Erfindung betrifft demzufolge auch Antikörper und Nukleinsäuren, die als Sonden bzw. Primer für solche Verfahren geeignet sind, und die Verwendung solcher Antikörper und Nukleinsäuren zur Diagnose und Analyse von Tumoren, insbesondere Magenkarzinomen.

Entsprechend betrifft die Erfindung bevorzugt auch Antikörper gegen Epitope, die von Exons v5 und/oder v6 kodiert werden, sowie Nukleinsäuren, die mit den Exons v5 und/oder v6 hybridisieren können, ferner Primer, die zur RT-PCR-Amplifikation von RNA, die die Exons v5 und/oder v6 enthält, verwendet werden können.

Zur *in-vivo*-Diagnostik können die CD44v-spezifischen Antikörper beispielsweise mit einem detektierbaren Marker, z. B. einem Radioisotop, verknüpft und in das Gefäßsystem eines Patienten injiziert werden. Nach der selektiven Bindung der Antikörper an den Tumor kann dieser mit einem geeigneten Detektionssystem visualisiert werden. Protokolle für solche immundiagnostischen Verfahren *in vivo* sind Stand der Technik (Thomas *et al.,* 1989).

Die Nuklein- und Aminosäuresequenz der varianten Exons des CD44-Gens ist bekannt (Hofmann *et al.,* 1991, Screaton *et al.,* 1992, Tölg *et al.,* 1993). Die Existenz degenerierter oder alleler Varianten ist für die Ausführung der Erfindung nicht von Bedeutung; solche Varianten sind daher ausdrücklich mit eingeschlossen.

Die Sequenz von Exon v5 des menschlichen CD44-Gens ist (SEQ ID NO: 1):

(D) V D R N G T T A Y E G N W
(CAG)AT GTA GAC AGA AAT GGC ACC ACT GCT TAT GAA GGA AAC TGG

N P E A H P P L I H H E H H E
AAC CCA GAA GCA CAC CCT CCC CTC ATT CAC CAT GAG CAT CAT GAG

E E E T P H S T S T
GAA GAA GAG ACC CCA CAT TCT ACA AGC ACA A.

Die Sequenz von Exon v6 des menschlichen CD44-Gens ist (SEQ ID NO: 3):

Q A T P S S T T E E T A T Q
TC CAG GCA ACT CCT AGT AGT ACA ACG GAA GAA ACA GCT ACC CAG

K E Q W F G N R W H E G Y R Q
AAG GAA CAG TGG TTT GGC AAC AGA TGG CAT GAG GGA TAT CGC CAA

T P R E D S H S T T G T A
ACA CCC AGA GAA GAC TCC CAT TCG ACA ACA GGG ACA GCT G.

Die erfindungsgemäßen Antikörper sind demzufolge vorzugsweise gegen Epitope innerhalb der dargestellten Aminosäuresequenzen oder deren allele Varianten gerichtet.

Besonders bevorzugt ist ein v6-spezifischer Antikörper, der an ein Epitop innerhalb der Aminosäuresequenz (SEQ ID NO: 10) QWFGNRWHEGYRQT oder einer allelen Variante dieser Sequenz bindet.

Besonders bevorzugt sind monoklonale Antikörper. Für das erfindungsgemäße Verfahren können jedoch auch andere Antikörpermoleküle verwendet werden, z.B. Fab- oder F(ab')₂-Fragmente von Immunglobulinen, rekombinant hergestellte single-chain-Antikörper (scFv), chimäre bzw. humanisierte Antikörper sowie andere Moleküle, die spezifisch an Epitope binden, die durch Exons v5 und/oder v6 kodiert werden. Die Herstellung von Antikörpern gegen bekannte Aminosäuresequenzen kann nach an sich bekannten Methoden erfolgen (Catty, 1989). Beispielsweise kann ein Peptid dieser Sequenz synthetisch hergestellt und als Antigen in einem Immunisierungsprotokoll eingesetzt werden. Ein anderer Weg ist die Herstellung eines Fusionsproteins, das die gewünschte Aminosäuresequenz enthält, indem eine Nukleinsäure (die synthetisch oder z.B. durch Polymerase-Kettenreaktion (PCR) aus einer geeigneten Probe hergestellt werden kann), die für diese Sequenz kodiert, in einen Expressionsvektor integriert und das Fusionsprotein in einem Wirtsorganismus exprimiert wird. Das gegebenenfalls gereinigte Fusionsprotein kann dann als Antigen in einem Immunisierungsprotokoll eingesetzt und Insert-spezifische Antikörper oder, im Falle monoklonaler Antikörper, Hybridome, die insertspezifische Antikörper exprimieren, mit geeigneten Verfahren selektiert werden. Solche Verfahren sind Stand der Technik. Heider *et al.* (1993) und Koopman *et al.* (1993) beschreiben die Herstellung von Antikörpern gegen variante Epitope von CD44.

Ebenfalls als Mittel zur Ausführung können Nukleinsäuren dienen, die mit varianten Exons, insbesondere v5 und/oder v6 hybridisieren, insbesondere solche mit einer Homologie von mehr als 80 % zu den entsprechenden Exonsequenzen. Die Herstellung solcher Nukleinsäuren kann nach an sich bekannten Methoden erfolgen. Dies gilt analog auch für Primer, die in der RT-PCR von RNA zum Nachweis der Expression der Exons v5 und/oder v6 verwendet werden können.

Die Ausführung des erfindungsgemäßen Verfahrens zur Diagnose und/oder Analyse von Tumoren, insbesondere von Magenkarzinomen, kann zweckmäßig durch Untersuchungen von aus dem Körper entnommenen Proben, beispielsweise aus Biopsien, erfolgen. Vorteilhaft können dabei die erfindungsgemäßen Mittel verwendet werden.

Beispielsweise können Gewebsschnitte immunhistochemisch mit den erfindungsgemäßen Antikörpern mit an sich bekannten Methoden untersucht werden. Aus Gewebeproben gewonnene Extrakte oder Körperflüssigkeiten können ferner mit anderen immunologischen Methoden unter Verwendung der besagten Antikörper untersucht werden, beispielsweise in Western Blots, Enzyme-linked Immunosorbent Assays (ELISA, Catty *et* Raykundalia, 1989), Radioimmunoassays (RIA, Catty *et* Murphy, 1989) oder verwandten Immunoassays.

Der Nachweis der Expression varianter Exons kann auch auf Nukleinsäureebene erfolgen, beispielsweise durch Hybridisierung von aus Gewebeproben gewonnener RNA (Northern Blot) oder RT-PCR-amplifizierter RNA mit geeigneten Primern, oder durch Hybridisierung von Nukleinsäuren in Gewebsschnitten mit geeigneten Proben *(in-situ*-Hybridisierung).

Die Untersuchungen können qualitativ, semiquantitativ oder quantitativ erfolgen.

Durch Nachweis und/oder Quantifizierung der Expression varianter CD44-Epitope erhobene Daten können so in die Diagnose und Prognose einfließen. Vorteilhaft kann dabei die Kombination mit anderen prognostischen Parametern sein, etwa mit der Gradierung.

Die erfindungsgemäßen Verfahren und Mittel eignen sich hervorragend zur Diagnose und/oder Analyse von Tumoren, insbesondere von Magenkarzinomen. Dies belegen die nachstehenden Beispiele. Die vergleichende Untersuchung der v5- und der v6-Expression erlaubt einen wesentlichen Beitrag zur Differentialdiagnose von intestinalem und diffusem Magenkarzinom.

### Abbildungen

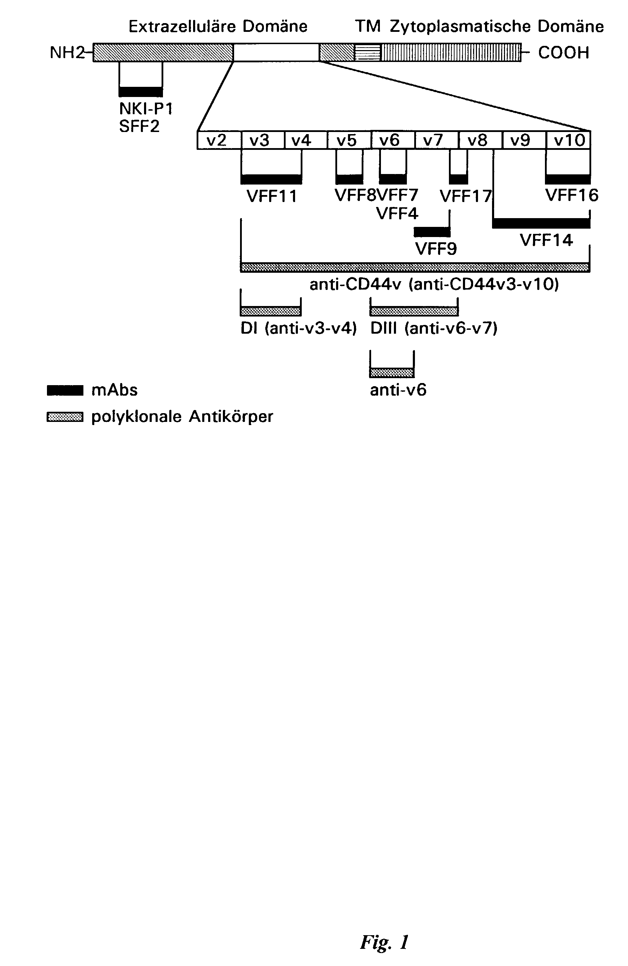

***Fig. 1:*** Schematische Darstellung eines CD44-Moleküls, das die varianten Exons v2 bis v10 enthält. Schraffierte Kästen symbolisieren CD44-Standardsequenzen (CD44s). TM = Transmembrandomäne. Die ungefähre Lokalisierung der Epitope, die von verschiedenen monoklonalen (schwarze Balken) und polyklonalen (graue Balken) Antikörpern erkannt werden, ist angezeigt. Die Antikörper VFF7, VFF8 und VFF16 sind jeweils für ein Exon spezifisch, wohingegen mAb VFF17 mit einem Epitop reagiert, daß von den Exons v7 und v8 gemeinsam kodiert wird.

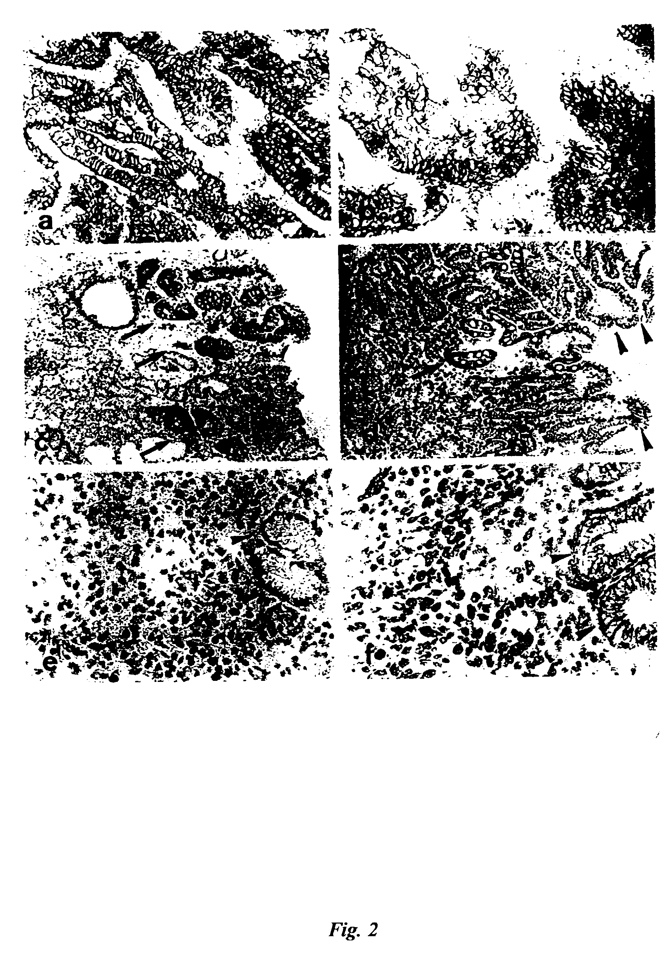

***Fig.* 2:** Immunhistochemie von normaler Mucosa sowie Adenokarzinomen des Magens. Eine fokal betonte anti-CD44v-positive Reaktion zeigt sich in Tumorzellen eines mäßig differenzierten Adenokarzinoms (intestinaler Typ nach Lauren) des Magens **(*a*)** sowie in einer regionalen Lymphknotenmetastase **(*b*)**. In normaler Mucosa des Magens mit chronischer Gastritis reagieren die Foci von intestinalen Metaplasien positiv mit mAb VFF4 (***c***, Pfeile) sowie mit mAb VFF8 *(**d**,* Pfeile), begleitet von einer zusätzlichen Reaktion auf der mucoiden Oberfläche und dem foveolären Epithel *(**d**,* Pfeilspitzen). Fast alle Becherzellkarzinome des Magens (diffuser Typ nach Lauren) zeigen eine negative Reaktion mit mAb VFF4 **(*e*)**, und im Gegensatz zu Adenokarzinomen des intestinalen Typs ist das normale mucoide Epithel negativ *(**e**,* Pfeilspitzen). In den meisten Fällen zeigt sich in diesen Becherzellkarzinomen eine positive Reaktion mit mAb VFF8 ***(f),*** und auch das verbliebene normale mucoide Epithel zeigt Immunreaktivität (***f***, Pfeilspitzen).
(ABC-Methode. ***(a)***, ***(b)***: anti-CD44v polyklonales Serum, 140 x; ***(c)***: VFF4, 80x; ***(d)***: VFF8, 80x; ***(e)***: VFF4, 210x; ***(f)***: VFF8, 210x; Gegenfärbung Hämatoxylin).

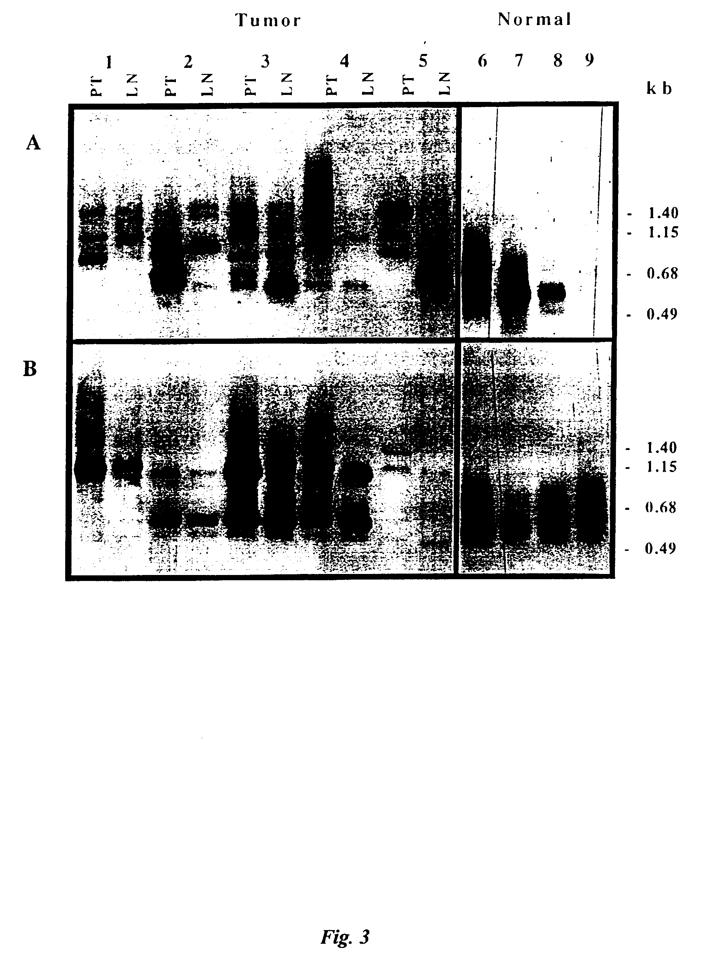

***Fig. 3:*** Southern-Blot-Analyse von PCR-Amplifikationsprodukten von einzelnen Proben von normaler gastrischer Mucosa, von primären Magentumoren und entsprechenden Lymphknotenmetastasen. Die PCR-Primer waren spezifisch für CD44-Exons, die den varianten Exonsequenzen benachbart sind. cDNAs wurden durch reverse Transkription erzeugt und vor der CD44-spezifischen Amplifikation durch GAPDH-PCR kontrolliert, um Qualität und Häufigkeit der synthetisierten cDNAs zu beurteilen. Die PCR-Produkte, die mit den CD44-Standardprimern erhalten wurden, wurden durch Elektrophorese in einem 1.2%igen Agarosegel aufgetrennt und auf Hybond N⁺-Membranen (Amersham, Braunschweig, Deutschland) übertragen. Die gleichen Filter wurden nacheinander mit Exon-v5-spezifischen (Positionen 243 bis 356 der publizierten menschlichen CD44v-Sequenz nach Hofinann *et al.,* 1991) ***(A)*** und Exon-v6-spezifischen (Positionen 360 bis 482) ***(B)*** Proben hybridisiert. *Spuren 1 bis 5:* 5 verschiedene primäre Adenokarzinome des Magens (PT) mit entsprechenden Lymphknotenmetastasen (LN), die in der Probensammlung, die immunhistochemisch untersucht wurde, nicht enthalten waren; *Spuren 1 bis 4:* intestinaler Typ; *Spur 5:* diffuser Typ, *Spuren 6 bis 9:* Normale gastrische Mucosa von vier verschiedenen Patienten aus der Korpus-(Spuren 6-8) und Antrumregion (Spur 9).

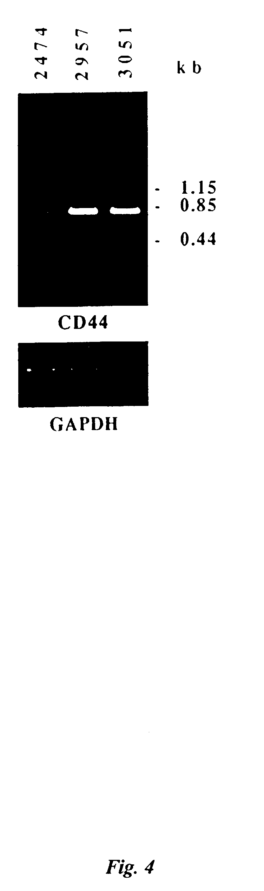

***Fig. 4:*** Reverse Transkription/PCR-Amplifikation von CD44-Transkripten in Magenkarzinom-Zellinien. Oberer Teil: PCR-Amplifikationsprodukte nach Benutzung von CD44-Standardprimern. Die Erststrangsynthese wurde mit polyadenylierter RNA aus den Magenkarzinomzellinien 2474, 2957 und 3051 durchgeführt. Unterer Teil: GADPH-spezifische PCR-Amplifikationsprodukte der gleichen RNA-Proben. Die Proben wurden in einem 1.2%igen Agarosegel aufgetrennt, mit Ethidiumbromid gefärbt und unter UV-Licht sichtbar gemacht.

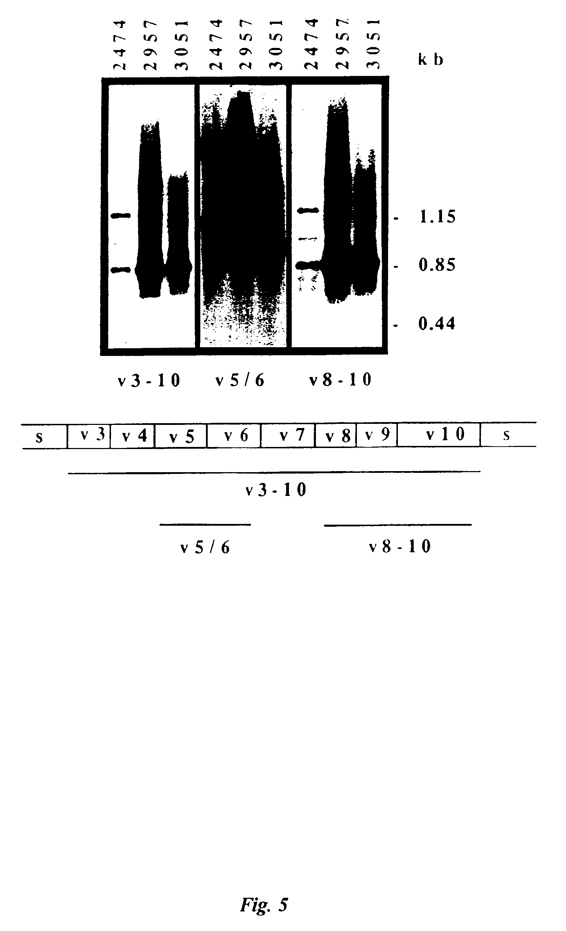

***Fig. 5:*** Southern-Blot-Analyse von CD44-PCR-Amplifikationsprodukten in Magenkarzinom-Zellinien. Die PCR-Reaktionen von Fig. 11 wurden in einem 1.2%igen Agarosegel aufgetrennt, auf eine Hybond N⁺-Membran übertragen und nacheinander mit Proben hybridisiert, die für verschiedene variante CD44-Exons spezifisch waren. v3-10 enthält Nukleotide von Position 25 bis 1013; v5/v6 enthält Nukleotide von Position 244 bis 468; v8-10 enthält Nukleotide von Position 623 bis 981 der veröffentlichten menschlichen varianten CD44-Exonsequenzen (Hofinann *et al.,* 1991).

Im folgenden wird die Eignung des erfindungsgemäßen Verfahrens zur Diagnose und/oder Analyse Magenkarzinomen demonstriert.

### Beispiele

### Beispiel 1: Herstellung von Antikörpern gegen Epitope, die von varianten Exonsequenzen des CD44-Gens kodiert werden

### 1.1. Klonierung von pGEX-Fusionsproteinen

Die gesamte variante Region des HPKII-Typs von CD44v (Hofinann *et al.,* 1991) wurde aus menschlicher Keratinozyten-cDNA durch Polymerase-Kettenreaktion (PCR) amplifiziert. Die beiden PCR-Primer *5*'-CAGGCTGGGAGCCAAATGAAGAAAATG-*3*' (SEQ ID NO: 11), Positionen 25-52, und *5*'-TGATAAGGAACGATTGACATTAGAGTT GGA-*3'* (SEQ ID NO: 12), Positionen 1013-984 der LCLC97-varianten Region, wie von Hofinann *et al.* beschrieben, enthielten eine *Eco*RI-Erkennungsstelle, die benutzt wurde, um das PCR-Produkt direkt in den Vektor pGEX-2T (Smith *et al.,* 1988) zu klonieren. Das resultierende Konstrukt (pGEX CD44v HPKII, v3-v10) kodiert für ein Fusionsprotein von ∼70 kD, bestehend aus Glutathion-S-transferase von *Schistosoma japonicum* und den Exons v3-v10 von humanem CD44 (Fig. 1; Heider *et al.,* 1993). Das Fusionsprotein wurde in *E. coli* exprimiert und anschließend über Glutathion-Agarose affinitätsgereinigt (Smith *et al.,* 1988).

Um Subklone der varianten Regionen zu erhalten, die für Affinitätsreinigungen und Western-Blot-Analysen verwendet werden konnten, wurden Fragmente kloniert, die DI (v3), DII/III (v5, v6), und DIII (v6, v7) enthielten, wobei die passenden Restriktionsschnittstellen verwendet wurden. Fusionsprotein DI enthält die CD44-Sequenz, die von Stamenkovic *et al.* (1989) beschrieben wurde, von Position 744, bis zur Position 142 der Sequenz von variantem CD44, wie sie von Hofinann *et al.* (1991) beschrieben wurde. Fusionsprotein DII/III enthält die variante Sequenz von Position 290-460, Fusionsprotein DIII die variante Sequenz von Position 378-638 (Hofinann *et al.,* 1991). Die DI und DIII enthaltenden Fragmente wurden in das pGEX-Vektorsystem, das DII/III-Fragment in den pATH-Vektor (Angel *et al.,* 1988) kloniert.

### 1.2. Monoklonale Antikörper: Immunisierung und Screening

Weibliche Balb/c Mäuse wurden intraperitoneal mit affinitätsgereinigtem Fusionsprotein (GST-CD44v3-10) immunisiert. Bei der 1. Immunisierung wurden 90 µg Fusionsprotein in komplettem Freund'schen Adiuvans, bei der 2. und 3. Immunisierung je 50 µg Fusionsprotein in inkomplettem Freund'schen Adjuvans appliziert. Die Immunisierungen erfolgten im Abstand von jeweils 4 Wochen. 14 Tage nach der letzten Immunisierung wurden die Tiere noch an drei aufeinanderfolgenden Tagen mit jeweils 10 µg Fusionsprotein in PBS immunisiert. Am darauffolgenden Tag wurden Milzzellen eines Tieres mit hohem Antikörpertiter mit P3.X63-Ag8.653-Mausmyelomzellen mit Hilfe von Polyethylenglycol 4000 fusioniert. Die Hybridomzellen wurden dann in Mikrotiterplatten in HAT-Medium selektioniert (Köhler *et* Milstein, 1975; Kearney *et al.,* 1979).

Die Bestimmung des Antikörpertiters im Serum bzw. das Screening der Hybridomüberstände wurde mit Hilfe eines ELISAs durchgeführt. Bei diesem Test werden zunächst Mikrotiterplatten mit Fusionsprotein (GST-CD44v3-10) oder nur mit Glutathion -S-transferase beschichtet. Anschließend wurde mit seriellen Verdünnungen von Serumproben bzw. Hybridomüberständen inkubiert und die spezifischen Antikörper mit Peroxidase-konjugierten Antikörpern gegen Maus-Immunglobulin nachgewiesen. Hybridome, die nur mit Glutathion-S-transferase reagierten, wurden verworfen. Die verbleibenden Antikörper wurden zunächst in einem ELISA mit domänenspezifischen Fusionsproteinen (Exon v3, Exon v5 + v6, Exon v6 + v7, Exon v8 - v10) charakterisiert (Koopman et al., 1993). Ihre immunhistochemische Reaktivität wurde an menschlichen Hautschnitten getestet.

Die Exonspezifität verschiedener Antikörper ist in Fig.1 dargestellt. VFF7 erkennt ein Epitop in der Aminosäuresequenz von Exon v6. VFF8 erkennt ein Epitop in der Aminosäuresequenz von Exon v5. VFF-17 erkennt ein Epitop, das von den Exons v7 und v8 gemeinsam kodiert wird. Die Antikörper NKI-P1 und SFF2 erkennen Epitope im Standardanteil der extrazellulären Domäne in der Nähe des N-Terminus.

### 1.3. Polyklonale Antikörper

Die Herstellung und Reinigung polyklonaler Antiseren gegen die variante Region des CD44-Moleküls ist in der Literatur beschrieben (Heider *et al.,* 1993). Die Exonspezifität des Gesamtserums (anti-CD44v) sowie affinitätsgereinigter Fraktionen (anti-DI, anti-DIII) ist in Fig. 1 angezeigt.

### Beispiel 2: Diagnose und Analyse von Magenkarzinomen

### 2.1. Tumoren und Gewebe

Tumorproben und Normalgewebe wurden aus den Beständen der Abteilung Pathologie der Universität Würzburg, Deutschland, ausgewählt. Die Proben waren unmittelbar nach der chirurgischen Entnahme schockgefroren und bis zur Verwendung bei -80°C gelagert worden. Normalgewebe wurde von zwölf verschiedenen Tumor-Patienten sowohl aus der Korpus- als auch aus der Antrumregion des Magens entnommen. Pathologische Gewebe wurden von einer Gesamtzahl von 47 Patienten mit einem Durchschnittsalter von 63 Jahren erhalten. Von den Primärkarzinomen gehörten 29 zum intestinalen und 18 zum diffusen Typ nach Lauren (1965). Die Tumorstadien reichten von lokalisiert (pT1) bis ausgedehnt (pT4), die histologische Gradierung von gut differenzierten (G1) bis schlecht differenzierten (G3) Adenokarzinomen.

### 2.2. Immunhistochemie von Magentumoren

Die Herstellung der verwendeten Antikörper kann Beispiel 1 entnommen werden.

Immunfärbungen wurden nach Standardprotokollen wie früher beschrieben durchgeführt (Heider *et al.,* 1993). Gefrierschnitte (6-7 µm) wurden in eisgekühltem Methanol 4 min., dann in Aceton 1 min. fixiert, in PBS (8 g/l NaCl, 0.2 g/l KCI, 1.44 g/l Na₂HPO₄, 0.24 g/l KH₂PO₄, pH 7.4) gewaschen und mit normalem Ziegenserum (10% in PBS) präinkubiert. Dann wurden sie 3x mit PBS gewaschen und für 1 Stunde mit dem Primärantikörper (in PBS, 1% BSA) inkubiert. Für monoklonale Antikörper wurde eine Endkonzentration von 5 µg/ml verwendet. Affinitätsgereinigte polyklonale Seren wurden auf normalen Hautkeratinozyten titriert, um optimale Färbungsergebnisse zu erhalten. Endogene Peroxidase wurde mit 0.3 % H₂O₂ in Methanol blockiert und die Schnitte mit biotinyliertem Zweitantikörper (entweder anti-Maus oder anti-Kaninchen F(ab')₂, DAKO Corp., Santa Barbara, CA, USA, abhängig vom verwendeten Primärantikörper) inkubiert. Der Immunkomplex wurde mit Meerrettich-Peroxidase visualisiert, die als Streptavidin-Biotin-Peroxidasekomplex an Biotin gekoppelt wurde (DAKO). Nach dreißigminütiger Inkubation mit dem Streptavidin-Biotin-Peroxidase-Komplex wurden die Schnitte mit 3,3-Amino-9-ethyl-carbazol (Sigma Chemicals, Deisenhofen, Deutschland) für 5 bis 10 min. entwickelt und die Reaktion mit H₂O abgestoppt. Die Zellen wurden mit Hämatoxylin gegengefärbt, mit Glyzerin-Gelatine eingedeckelt und mikroskopisch untersucht.

Polyklonale Antiseren, die gegen die Epitope v3-v10 gerichtet sind (CD44v in Fig. 1), färbten 42/42 Gefrierschnitte von Magentumoren (Tabelle 1, Beispiele in Fig. 2a,b). Die Färbung war heterogen im Hinblick auf Intensität und Verteilung. Zwischen 5% und 100% der Tumorzellen waren mit unterschiedlicher Intensität angefärbt. Dies bestätigt den Befund von Heider *et al.* (1993), daß Adenokarzinome CD44-Spleißvarianten exprimieren. Überraschend zeigten die nachfolgenden Untersuchungen jedoch, daß sich die Feinanalyse des Expressionsmusters der verschiedenen varianten Exons zur Diagnose und/oder Analyse von Magenkarzinomen eignet. Dabei sind sowohl qualitative als auch semiquantitative Schlußfolgerungen möglich.

Gefrierschnitte von Tumoren wurden mit exonspezifischen monoklonalen Antikörpern (mAbs) untersucht. Fast alle Tumoren, die positiv mit dem polyklonalen Antiserum reagierten, reagierten ebenso positiv mit einem mAb gegen ein Exon-v5-spezifisches Epitop (VFF8; Tabelle 1; ein Beispiel zeigt Fig. 2f). Im Gegensatz dazu war die Reaktion mit dem v6-spezifischen Antikörper VFF4 viel stärker eingeschränkt, nur 26/42 Tumoren reagierten positiv (Tabelle 1). Mabs, die andere Exons erkannten (v3/4, v7, v8-10), banden nicht (Tabelle 1).

Überraschenderweise waren 23 der 26 VFF4-positiven Tumoren Adenokarzinome des intestinalen Typs, während 14 der 16 v6-negativen Fälle Siegelringkarzinome des diffusen Typs waren (ein Beispiel ist in Fig. 2c gezeigt). Die vorliegende Erfindung stellt somit erstmalig einen molekularen Marker zur Unterscheidung verschiedener Subtypen von Magenkarzinomen bereit.

Von 10 Patienten waren sowohl Primärtumoren als auch Lymphknotenmetastasen erhältlich. Fünf dieser Paare gehörten zum intestinalen Typ und fünf zum diffusen Typ. Epitope, die durch das polyklonale Antiserum erkannt wurden, waren sowohl auf den Primärtumoren als auch auf den Metastasen von allen 10 Tumorpaaren präsent (Tabelle 2; Fig. 2a und b zeigen den Primärtumor Nr. 9069/90 von Tabelle 2 und die zugehörige Lymphknotenmetastase). Alle Tumorproben (Primärtumoren und Metastasen) reagierten mit dem für Exon v5 spezifischen mAb VFF8. Alle Proben, die zum intestinalen Typ gehören, reagierten positiv nach Inkubation mit dem für Exon v6 spezifischen mAb VFF4, währen von den Siegelringkarzinomen nur ein Paar zur VFF4⁺-Gruppe gehörte (vgl. Tabelle 2). Es wurden keine Unterschiede in der Färbungsintensität zwischen Primärtumoren und Metastasen entdeckt, noch gab es konsistente Unterschiede im Anteil von CD44v-positiven Zellen zwischen Primärtumor und Metastasen (Tabelle 2). Dies trifft insbesondere auf die Lymphknotenmetastasen der VFF4(Exon v6)-negativen Siegelringkarzinome des diffusen Typs zu, die ebenfalls nicht mit diesem mAb reagierten (Tabelle 2). Die vorliegende Erfindung stellt somit auch erstmalig ein Verfahren sowie die Mittel zu seiner Durchführung bereit, mit dem durch Analyse von Metastasen anhand molekularer Marker direkte Rückschlüsse auf den Primärtumor eines Adenokarzinoms möglich sind.

Um zu untersuchen, ob die Expression von CD44v in Adenokarzinomen, insbesondere des Magens, ein Ergebnis des Transformationsprozesses ist, oder ob CD44 bereits im Normalgewebe, z.B. des Gastrums, exprimiert wird, wurden Gefrierschnitte von normaler gastrischer Mucosa von zwölf verschiedenen Patienten durch immunhistochemische Färbung mit für CD44-Varianten spezifischen Antikörpern analysiert. Alle zwölf Proben färbten positiv mit dem polyklonalen Serum sowie mit einigen der monoklonalen Antikörper. Mit dem polyklonalen Antiserum (Exons v3-v10) und dem mAb VFF8 (Exon-Sequenz v5) wurden positive Reaktionen auf dem mucoiden Oberflächenepithel, in der foveolären Proliferationszone und in Arealen von intestinaler Metaplasie erhalten (ein Beispiel ist in Fig. 9d gezeigt). Interessanterweise reagierten diese letzteren Areale auch positiv mit VFF4 (Exon-Sequenz v6), während andere Teile des normalen Magenepithels nicht mit diesem mAb reagierten (Fig. 2c). Alle anderen mAbs (VFF11, VFF9, VFF14; Epitopspezifität vgl. Fig. 1) reagierten nicht. In bestimmten Bereichen der Magenmucosa wird also eine Spleißvariante von CD44 exprimiert, die die Exonsequenz v5 trägt, und die deshalb in der Expression den Zellen von Karzinomen des diffusen Typs ähnelt. Bereiche der Magenmucosa, die durch intestinale Metaplasien gekennzeichnet sind, tragen dagegen Epitope beider Exons v5 und v6 und sind in der Expression daher Karzinomen vom intestinalen Typ ähnlich. Diese Befunde unterstützen die These, daß die Tumoren von diesen jeweiligen Zelltypen abstammen und das Expressionsmuster der Zellen aufrechterhalten, von denen sie abstammen. Die vorliegende Erfindung macht solche Analysen von Adenokarzinomen, insbesondere des Magens, erstmalig möglich.

**Tabelle 1:**

| Expression von varianten CD44-Epitopen auf den Zelloberflächen von Magentumoren | | | | | | |
|---|---|---|---|---|---|---|
| | Serum¹ | Monoklonale Antikörper | | | | |
| Adenokarzinome | αCD44v | VFF11 | VFF8 | VFF4 | VFF9 | VFF14 |
| | (v3-v10) | (v3/4) | (v5) | (v6) | (v7) | (v8-10) |
| Diffuser Typ | 17/17² | 0/17 | 14/17 | 3/17 | 0/17 | 0/17 |
| Intestinaler Typ | 25/25 | 0/25 | 25/25 | 23/25 | 0/25 | 0/25 |
| Gesamt | 42/42 | 0/42 | 39/42 | 26/42 | 0/42 | 0/42 |

| | | | | | | |
|---|---|---|---|---|---|---|
| ¹ polyklonales Antiserum | | | | | | |
| ² Zahl positiver Tumoren/Zahl der untersuchten Tumoren | | | | | | |

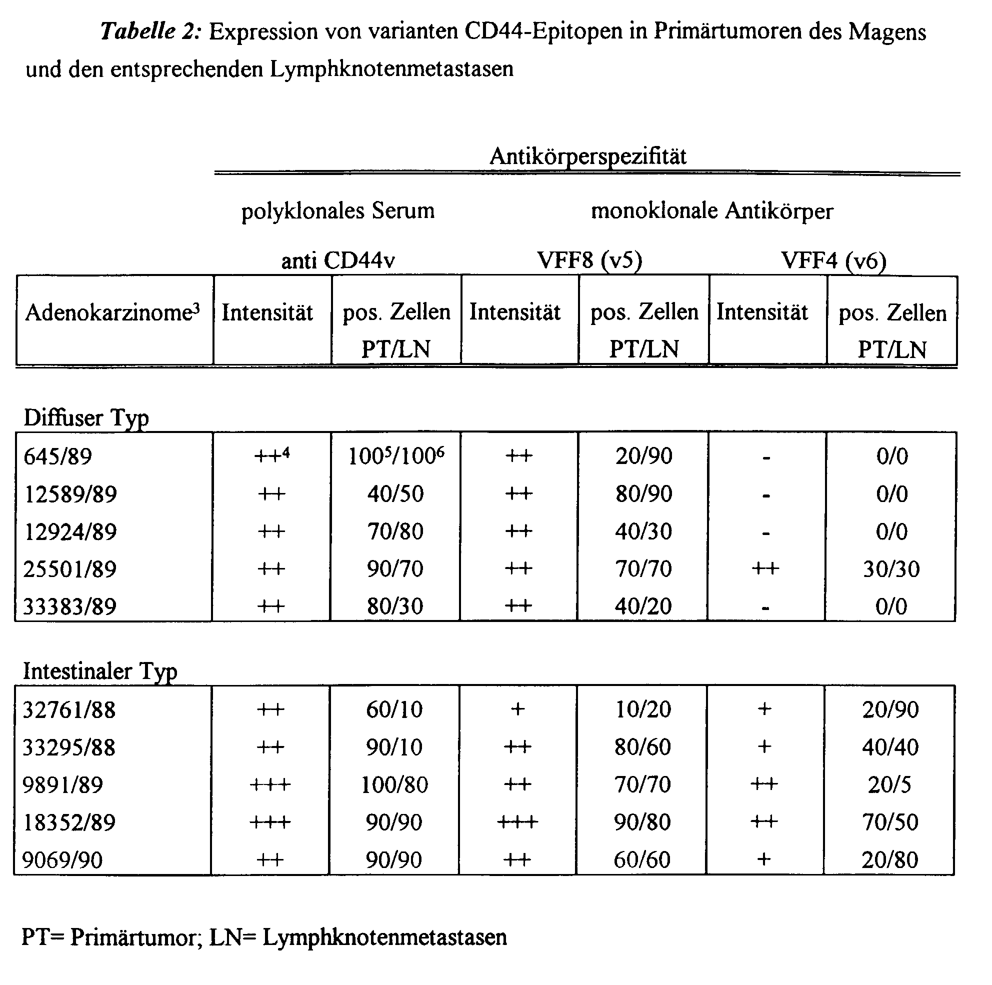

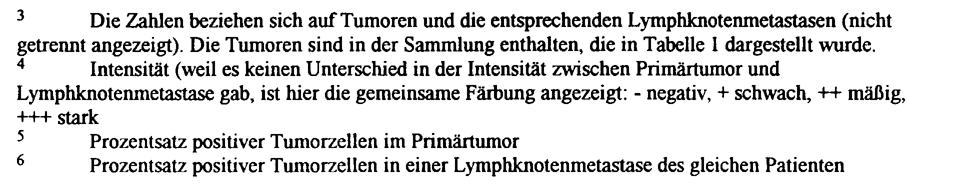

### 2.3 Western-Blot-Analyse

Zellen wurden durch Ultraschall lysiert und in SDS-Gel-Probenpuffer 3 min. gekocht (Lämmli, 1970). Gleiche Proteinmengen (ermittelt durch Färbung des Gels mit Coomassie Brilliant Blue) wurden elektrophoretisch in einem denaturierenden 6%igen Polyacrylamidgel aufgetrennt (Lämmli, 1970). Die Proteine wurden auf Polyvinyliden Difluorid-Membran (Millipore, Eschborn, Deutschland) übertragen, wobei ein Transblot-Gerät (BIO-RAD Laboratories, München, Deutschland) verwendet wurde. Unspezifische Wechselwirkungen wurden durch Präinkubation der Membranen mit einer Milchpulversuspension (10% Trockenmilchpulver in PBS) blockiert. Die Membranen wurden dann bei Raumtemperatur mit dem polyklonalen anti-CD44-Antiserum (vgl. Fig. 1) inkubiert, gefolgt von einer weiteren Inkubation mit mit alkalischer Phosphatase konjugiertem Ziegen-anti-Kaninchen-IgG (Amersham, Braunschweig, Deutschland). Die Inkubationszeit betrug jeweils eine Stunde. Nach jeder Antikörperinkubation wurden die Membranen mit PBS gewaschen, das 0.3% Tween 20 (Sigma) enthielt. Die Bindung der Antikörper wurde unter Verwendung eines Enhanced Chemoluminiscent System (Amersham) nachgewiesen.

### 2.4. Reverse Transkription /PCR-Amplifikation

Um zu untersuchen, ob die Expressionsmuster der Spleißvarianten in normalem Gewebe und in Tumorgewebe gleich oder unterschiedlich sind, wurde RNA von Normalgewebe und aus Tumoren isoliert, revers transkribiert, durch PCR amplifiziert und mit Exon-spezifischen Proben hybridisiert. Für die PCR wurden Primer benutzt, die auf der 5'-bzw. 3'-Seite der Insertionsstelle der varianten Exons lokalisiert sind.

2 µg Gesamt-RNA (aus Geweben) oder polyA⁺-RNA (aus Zellinien) wurden isoliert und revers transkribiert, wie in der Literatur beschrieben (Günthert *et al*., 1991). 5 µl der Erststrang-cDNA wurden mit *Taq*-Polymerase (Amersham) in 50 µl unter den vom Hersteller empfohlenen Pufferbedingungen amplifiziert. Für die GAPDH-PCR wurden Oligonukleotide verwendet, die zu den Positionen 8-29 und 362-339 der publizierten cDNA-Sequenz (Allen *et al*., 1987) homolog waren. Nach 26 Amplifikationsrunden (95°C für 1 min., 62°C für 1 min., 72°C für 2.5 min.) wurden 10 µl des Reaktionsansatzes in einem 1.4%igen Agarosegel aufgetrennt und das Amplifikationsprodukt nach Färbung mit Ethidiumbromid unter UV-Licht visualisiert. Zur Amplifikation der varianten CD44-Transkripte wurden Primer verwendet, die homolog zu den Positionen 513-540 (5'-oligo) und 900-922 (3'-oligo) der publizierten menschlichen CD44-Sequenz (Stamenkovic *et al.,* 1989) waren. Nach 36 Amplifikationsrunden (94°C für 1 min., 62°C für 1 min., 72°C für 2.5 min.) wurden 10 µl des Reaktionsansatzes in einem Agarosegel aufgetrennt, nach Färbung des Gels mit Ethidiumbromid unter UV-Licht visualisiert und anschließend auf Nylon-Membranen für die Durchführung der Southern Blots (vgl. Fig. 3 und 5 sowie die zugehörigen Abbildungstexte) nach Standardprotokollen (Sambrook *et al.,* 1985) übertragen.

Durch Ethidiumbromidfärbung von Agarosegelen wurde sowohl in Proben aus normaler Mucosa als auch in Tumorproben ein dominantes PCR-Produkt detektiert, das nach seiner Größe der Form CD44s entsprach. PCR-Produkte geringerer Prominenz wurden nach Übertragung auf Nylonmembranen und Hybridisierung mit Exon-spezifischen Proben nachgewiesen (Fig. 3). Durch Hybridisierung mit einer v5-spezifischen bzw. einer v6-spezifischen Probe wurde die Expression von Exon v5 bzw. v6 enthaltender RNA nachgewiesen. Es ergab sich eine deutliche Differenz im Muster der RNA, die in normalem Gewebe und in Tumoren exprimiert wurde. Die RNA-Muster unterschieden sich ferner von Tumor zu Tumor.

RNA von 10 der 12 Proben von normaler Magenmucosa, die auch in der Immunhistochemie untersucht worden waren, ergab zwei dominante Banden, die mit Exons v6 hybridisierten, und zwei dominante Banden ähnlicher Größe, die mit Exon v5 hybridisierten (vier repräsentative Beispiele sind in Fig. 3A und B gezeigt). RNA aus Tumorproben ergab ein komplexeres und variableres Muster der Spleißprodukte. Die Tumoren enthielten zumindest teilweise auch größere Spleißvarianten bis zu einer Größe, die die Anwesenheit aller varianten Exonsequenzen vermuten läßt (Fig. 3). Offensichtlich waren nicht alle der durch diese Exons kodierten Epitope der Immunhistochemie zugänglich. Sowohl Tumoren vom diffusen als auch vom intestinalen Typ zeigten starke Expression von Transkripten, die Exon v5 enthielten (Fig. 3A, Spuren 1-5). Allerdings gab es einen klaren Unterschied zwischen Tumoren vom diffusen und vom intestinalen Typ, was die Hybridisierung mit der Exon-v6-spezifischen Probe betraf. Während in allen vier Proben vom intestinalen Typ Amplifizierungsprodukte nachgewiesen werden konnten, die Exon v6 enthielten (vgl. Fig. 3B, Spuren 1-4), zeigte die Probe eines Tumors vom diffusen Typ fast keine Hybridisierung mit der v6-spezifischen Probe (Fig. 3B, Spur 5). Die vorliegende Erfindung gestattet somit Diagnose und Analyse von Adenokarzinomen anhand molekularer Marker auf Nukleinsäureebene, und stellt die Mittel dafür zur Verfügung. Insbesondere gestattet das erfindungsgemäße Verfahren die Unterscheidung von Magenkarzinomen vom diffusen und vom intestinalen Typ sowie die Analyse der entsprechenden Metastasen auf Nukleinsäureebene.

### Literatur

Allen, R.W., Trach, K.A., and Hoch, J.A. Identification ofthe 37kDa protein displaying a variable interaction with the erythroid cell membrane as glyceraldehyde-3-phosphate dehydrogenase. *J. Biol. Chem. 262:* 649-652 (1987).

Angel P., Allegretto, E.A., Okuio, S.T.Hatton, K., Boyle, W.J., Hunter, T., Karin, M. Oncogene *jun* encodes a sequence-specific trans-activator similar to AP-1. *Nature 332:* 166 (1988).

Arch, R., Wirth, K., Hofinann, M., Ponta, H., Matzku, S., Herrlich, P., and Zöller, M. Participation in normal immune responses of a splice variant of CD44 that encodes a metastasis-inducing domain. *Science 257:* 682-685 (1992).

Brady, L.W., Perez, C.A., Bedwinnek, J.M. Failure patterns in gynecologic cancer. *Int. J. Rad. Oncol. Biol. Phys. 12:* 549-557 (1986).

Catty, D (Hrsg). *Antibodies Vols. I und II.* IRL Press Oxford (1989).

Catty, D., Raykundalia, C. ELISA and related immunoassays. In: Catty, D (Hrsg). *Antibodies Vol. II.* IRL Press Oxford (1989), 97-152, s. S. 105-109.

Catty, D., Murphy, G. Immunoassays using radiolabels. In: Catty, D (Hrsg). *Antibodies Vol. II.* IRL Press Oxford (1989), 77-96.

Diehl, I. J., Kaufmann, M., Goerner R., Costa, S. D., Kaul, S., Bastert, G. Detection of tumor cells in bone marrow ofpatients with primary breast cancer: A prognostic factor for distant metastasis. *J. Clin. Oncol. 10:* 1534-1539 (1992).

Fisher, E. R., Redmond, C., Fisher, B., Bass, G. Pathologic findings from the National Surgical Adjuvant Breast and Bowel Projects (BSABP). Prognostic discriminants for 8-year survival for node-negative invasive breast cancer patients. *Cancer 65:* 2121-2128 (1990).

Günthert, U., Hofinann, M., Rudy, W., Reber, S., Zöller, M., Haußmann, I., Matzku, S., Wenzel, A., Ponta, H., and Herrlich, P. A new variant of glycoprotein CD44 confers metastatic potential to rat carcinoma cells. *Cell 65:* 13-24 (1991).

Heider, K.-H., Hofmann, M., Horst, E., van den Berg, F., Ponta, H., Herrlich, P., and Pals, S.T.A human homologue ofthe rat metastasis-associated variant of CD44 is expressed in colorectal carcinomas and adenomatous polyps. *J. Cell Biol. 120:* 227-233 (1993).

Hofmann, M., Rudy, W., Zöller, M., Tölg, C., Ponta, H., Herrlich P., and Günthert, U. CD44 splice variants confer metastatic behavior in rats: homologous sequences are expressed in human tumor cell lines. *Cancer Res. 51:* 5292-5297 (1991).

Jass, J.R.A classification of gastric dysplasia. *Histopath. 7: 181-193* (1983).

Jida, F., and Kusama, J. Gastric and intestinal metaplasia. Significance oftype ofintestinal metaplasia upon development of gastric carcinoma. *Cancer 50:* 2854-2858 (1982).

Kato, Y., Kiagawa T., Nakamura, K., and Sugano, H. Changes in the histologic tpyes of gastric carcinoma in Japan. *Cancer 48:* 2084-2087 (1981).

Kearney, J.F., Radbruch A., Liesegang B., Rajewski K. A new mouse myeloma cell line that has lost imunoglobulin expression but permits construction of antibody-secreting hybrid cell lines. *J. Immunol. 123:* 1548 (1979).

Köhler, G., Milstein, C. Continous culture of fused cells secreting antibody ofpredefined specifity. *Nature 265:* 495 (1975)

Koopman, G., Heider, K.-H., Horts, E., Adolf, G. R., van den Berg, F., Ponta, H., Herrlich, P., Pals, S. T. Activated human lymphocytes and aggressive Non-Hodgkin's lymphomas express a homologue ofthe rat metastasis-associated variant of CD44. *J. Exp. Med*. *177:* 897-904 (1993).

Laemmli, U. Cleavage ofstructural proteins during the assembly of the head of bacteriophage T4. *Nature 227:* 680-685 (1970).

Lauren P. The two histological main types of gastric carcinoma: diffuse and so-called intestinal-type carcinoma. *Acta Path. Microbiol. Scand. 64:* 31-49 (1965).

Mackay, C. R., Terpe, H.-J., Stauder, R., Marston, W. L., Stark, H., Günthert U. Expression and modulation of CD44 variant isoforms in humans. *J. Cell Biol. 124:* 71-82 (1994).

Mayer, B., Jauch, K.W., Günthert, U., Figdor, C.G., Schildberg, F.W., Funke, I., Johnson, J.P. De-novo expression ofCD44 and survival in gastric cancer. *Lancet 342:* 1019-1022 (1993).

Nomura, A., Stemmermann, G.N., Chyon, P.H., Kato, I., Perez-Perez, G.I. and Blaser, H.J. *Helicobacter pylori* infection and gastric carcinoma among Japanese americans in Hawaii. *New Engl. J. Med. 325:* 1132-1136 (1991).

Park, R.C., Thigpen, J.T. Chemotherapy in advanced and recurrent cervical cancer. *Cancer 71:* 1446-1450 (1993).

Parsonnet, J., Friedman, G.D., Vandersteen, D.F., Chang, Y., Vogelman, J.H., Orentreich, N., and Sikley, R.K. Helicobacter pylori infection and the risk of gastric carcinoma. *New Engl. J. Med*. *325:* 1127-1131(1991)*.*

Perez, C.A., Breaux, S., Madoo-Jones, H., Bedwinek, J.M., Camel, H.M., Purdy, J.A., Walz, B.J. Radiation therapy alone in the treatment ofcarcinoma ofuterine Zervix. *Cancer 51:* 1393-1402 (1983).

Petterson, F. Annual report on results oftreatment in gynecologic cancer. *FIGO Cancer Comitees: Vol. 20 (1988).*

Piper, D.W. Stomach cancer. In: "Geneva: International Union Against Cancer". *UICC Technical Report Series Vol. 34* (1978).

Sambrook, J., Fritsch E.E., Maniatis I., *Molecular cloning.* Cold Spring Harbor Laboratory Press, Cold Spring Harbor (1989).

Screaton, G.R., Bell, M.V., Jackson, D.G., Cornelis, F.B., Gerth, U., and Bell, J. I. Genomic structure of DNA encoding the lymphocyte homing receptor CD44 reveals at least 12 alternatively spliced exons. *Proc. Natl. Acad*. *Sci. U.S.A. 89:* 12160-12164 (1992).

Sipponen, P., Kekki, M., and Surala, M. Atrophic chronic gastritis and intestinal metaplasia in gastric carcinoma. Comparison with a representative population sample. *Cancer 52:* 1062-1068 (1983).

Siurala, M., Lehtola, J., and Ihamäki, T. Atrophic gastritis and its sequelae. Results of 15-23 years follow-up. *Scand. J. Gastroenterol. 1:* 40-48 (1974).

Smith, D.B., Johnson, K.S. Single-step purification of polypetides expressed in *Escherichia coli* as fusions with glutathione S-transferase. *Gene 67:* 31 (1988).

Stamenkovic, I., Amiot, M., Pesando, J.M., and Seed, B.A lymphocyte molecule implicated in lymph node homing is a member ofthe cartilage link protein family. *Cell 56*: 1057- 1062 (1989).

Strickland, R.G., and Mackay, I.R.A reappraisal ofthe nature and significance ofchronic atrophic gastritis. *Dig. Dis. Sci. 18:* 426-440 (1973).

Tölg, C., Hofinann, M., Herrlich, P., and Ponta, H. Splicing choice from ten variant exons establishes CD44 variability. *Nucleic Acids. Res. 21:* 1225-1229 (1993).

Thomas, G. D., Dykes, P. W., Bradwell, A. R. Antibodies for tumour immunodetection and methods for antibody radiolabeling. In: Catty, D. (Hrsg.). *Antibodies Vol. II.* IRL Press Oxford , 223-244 (1989).

## Patentansprüche

1. Verfahren zur Differentialdiagnose und/oder Analyse von Magenkarzinomen diffusen oder intestinalen Typs *in vitro,* dadurch gekennzeichnet, daß es auf dem Nachweis der Expression der variablen Exons v5 und/oder v6 des CD44-Gens beruht.

2. Verfahren nach Anspruch 1, dadurch gekennzeichnet, daß der Nachweis ein immunologischer Nachweis ist.

3. Verfahren nach Anspruch 1, dadurch gekennzeichnet, daß es auf dem Nachweis einer Nukleinsäure beruht, die spezifisch für Exon v5 und/oder v6 ist.

4. Antikörper gegen variantes CD44, dadurch gekennzeichnet, daß er gegen ein Epitop gerichtet ist, das von Exon v5 kodiert wird.

5. Antikörper nach Anspruch 4, dadurch gekennzeichnet, daß das Epitop in der Aminosäuresequenz SEQ ID NO: 2, DVDRNGTTAYEGNWNPEAHPPLIHHEHHEEEETPHSTST enthalten ist.

6. Antikörper nach einem der Ansprüche 4 oder 5, dadurch gekennzeichnet, daß er monoklonal ist.

7. Antikörper nach einem der Ansprüche 4 bis 6, dadurch gekennzeichnet, daß er ein Fab- oder F(ab')₂-Fragment eines Immunglobulins, ein rekombinant hergestellter single-chain-Antikörper (scFv) oder ein chimärer bzw. humanisierter Antikörper ist.

8. Antikörper gemäß einem der Ansprüche 4 bis 7 zur diagnostischen Verwendung.

9. Verwendung eines Antikörpers gemäß einem der Ansprüche 4 bis 7 in einem Verfahren gemäß einem der Ansprüche 1 oder 2.

10. Verwendung eines Antikörpers gemäß einem der Ansprüche 4 bis 7 zur Herstellung eines Mittels für die Diagnose und/oder Analyse von Magenkarzinomen.

11. Verwendung eines Antikörpers, der gegen das variante Exon v6 des CD44-Gens gerichtet ist, in einem Verfahren gemäß einem der Ansprüche 1 oder 2.

12. Verwendung eines Antikörpers, der gegen das variante Exon v6 des CD44-Gens gerichtet ist, zur Herstellung eines Mittels für die Diagnose und/oder Analyse von Magenkarzinomen.

13. Verwendung einer Nukleinsäure in einem Verfahren gemäß Anspruch 3.
